# EUROPEAN PATENT APPLICATION

(11) **EP 3 360 466 A1**
(43) Date of publication of application: **15.08.2018**
(21) Application number: 17155212.8
(22) Date of filing: 08.02.2017
(51) Int. Cl.: A61B 5/00, A61B 5/11, A61B 5/16, G08B 23/00, G08B 13/14

(54) **A METHOD AND APPARATUS FOR MONITORING A SUBJECT**

(71) Applicant: Koninklijke Philips N.V., 5656 AE Eindhoven (NL)
(72) Inventor: Christiaan, VAREKAMP, 5656 AE Eindhoven (NL); Murtaza, BULUT, 5656 AE Eindhoven (NL)
(74) Representative: de Haan, Poul Erik

(57) **Abstract**

There is provided a method and apparatus for monitoring a subject. A sequence of sensor signals associated with an environment of the subject is acquired from at least one sensor signal. The acquired sequence of sensor signals is compared to at least one stored sequence of sensor signals. The stored sequence of sensor signals comprise one or more features associated with an activity. A stored sequence of sensor signals comprising one or more features that match features of the acquired sequence of sensor signals is identified. It is detected whether the subject performs the activity associated with the one or more features of the identified sequence of sensor signals. A failure of the subject to perform the activity is indicative of a memory loss event. A notification is output where the subject fails to perform the activity.

## Description

### Technical Field of the Invention

The invention relates to the field of monitoring of a subject and, in particular, to a method and apparatus for monitoring a subject, which can be particularly useful where the subject suffers from memory loss.

### Background to the Invention

The monitoring of a subject has become common practice in many situations. One such situation is the monitoring of subjects that may suffer from memory loss. Memory loss can be caused by dementia from which a growing number of people suffer. People with dementia often have difficulty performing day-to-day tasks. An example of dementia is Alzheimer's disease. There are several stages of Alzheimer's disease. The early stages symptoms include the person forgetting the location of objects. A middle-stage symptoms includes the person forgetting their own location and a risk of the person wandering.

An existing solution to help cope with memory loss is a wearable digital camera called the SenseCam. This wearable digital camera automatically takes images while the subject is wearing the camera without user intervention and the images that are acquired can be played back to review the daily activities of the subject. There exist systems that use the acquired images to perform automatic content analysis to group images by events, suggest their relative importance, and select representative images for reference. The details of a recorded experience can be optimised in this way to select cues to support episodic memory. An example of this type of optimisation is provided in "Providing Good Memory Cues for People with Episodic Memory Impairment", Matthew L. Lee & Anind K. Dey, Human-Computer Interaction Institute, 2007.

Other systems that can support memory loss are systems for localising and tracking objects. Existing systems that localise objects require that the objects are manually tagged such as using near-field communication (NFC) or radio-frequency identification (RFID) tags. Some systems use camera sensors to track objects and person-object interactions.

However, while the existing systems may be able to detect person-object interactions, the existing systems are unable to detect the failure of a memory loss event where a person intends to perform a certain task but subsequently forgets. These situations, in which a person experiences memory failure and is unable to complete a task that they intended to perform, can happen many times over a long time period without a caregiver noticing that memory failure is occurring. As such, many of the early warning signs for memory loss can go undetected.

There is thus a need for an improved method and apparatus for monitoring a subject.

### Summary of the Invention

As noted above, the limitation with existing approaches is that situations in which a person experiences memory failure and is unable to complete a task that they intended to perform can go unnoticed, leaving the early warning signs for memory loss undetected. It would thus be valuable to have a method and apparatus for monitoring a subject, which overcomes these existing problems.

Therefore, according to a first aspect of the invention, there is provided a method for monitoring a subject. The method comprises acquiring, from at least one sensor, a sequence of sensor signals associated with an environment of a subject and comparing the acquired sequence of sensor signals to at least one stored sequence of sensor signals, wherein the stored sequence of sensor signals comprise one or more features associated with an activity. The method also comprises identifying a stored sequence of sensor signals comprising one or more features that match features of the acquired sequence of sensor signals, detecting whether the subject performs the activity associated with the one or more features of the identified sequence of sensor signals, wherein a failure of the subject to perform the activity is indicative of a memory loss event, and outputting a notification where the subject fails to perform the activity.

In some embodiments, the sensor signals may comprise any one or more of visual signals, acoustic signals and motion signals.

In some embodiments, the features may comprise any one or more of an acquired sensor signal, a low-level feature characterising one or more aspects of the sensor signal, an object in the environment of the subject, a location of the subject in the environment, a movement of the subject in the environment, a posture of the subject in the environment, a time of day in the environment of the subject, the weather in the environment of the subject, and the number of people in the environment of the subject. In some embodiments, the one or more features of the stored sequence of sensor signals may each be stored with any one or more of a weight indicative of the significance of the feature compared to the other features, and an a priori probability of the subject failing to perform the activity associated with the one or more features.

In some embodiments, the notification is output to any one or more of the subject to assist the subject in performing the activity and another user to indicate the subject failing to perform the activity.

In some embodiments, the one or more features of the stored sequence of sensor signals may be associated with a risk level for the subject and the notification that is output may be based on the risk level associated with the one or more features of the identified sequence of sensor signals.

In some embodiments, the method may further comprise acquiring from at least one physiological sensor one or more physiological signals for the subject and determining a likelihood that the failure of the subject to perform the activity is due to the memory loss event based on the acquired physiological signals.

In some embodiments, the method may further comprise determining a severity of one or more memory loss events over time.

In some embodiments, the method may further comprise detecting whether the subject is moving to perform at least part of the activity and, where the subject fails to move to perform at least part of the activity for a minimum period of time, outputting the notification to a user other than the subject.

In some embodiments, the method may further comprise storing the at least one sequence of sensor signals in a calibration phase, wherein the one or more features of the at least one stored sequence of sensor signals may be indicative of any one or more of a location of the subject in the environment that is associated with the activity and one or more objects in the environment that are associated with the activity.

According to a second aspect of the invention, there is provided a computer program product comprising a computer readable medium, the computer readable medium having computer readable code embodied therein, the computer readable code being configured such that, on execution by a suitable computer or processor, the computer or processor is caused to perform the method or the methods described above.

According to a third aspect of the invention, there is provided an apparatus for monitoring a subject, the apparatus comprises a processor. The processor is configured to acquire from at least one sensor a sequence of sensor signals associated with an environment of a subject and compare the acquired sequence of sensor signals to at least one stored sequence of sensor signals, wherein the stored sequence of sensor signals comprise one or more features associated with an activity. The processor is also configured to identify a stored sequence of sensor signals comprising one or more features that match features of the acquired sequence of sensor signals, detect whether the subject performs the activity associated with the one or more features of the identified sequence of sensor signals, wherein a failure of the subject to perform the activity is indicative of a memory loss event, and output a notification where the subject fails to perform the activity.

In some embodiments, the at least one sensor may comprise any one or more of a visual sensor, an acoustic sensor, a motion sensor, an electromagnetic field, and a radiation sensor.

In some embodiments, the apparatus may comprise one or more of the at least one sensors. In some embodiments, a wearable device configured to be worn by the subject may comprise one or more of the at least one sensors, an object in the environment of the subject may comprise one or more of the at least one sensors, and/or one or more of the at least one sensors may be located in the environment of the subject.

According to the aspects and embodiments described above, the limitations of existing techniques are addressed. In particular, according to the above-described aspects and embodiments, it is possible to provide a notification when a person experiences memory failure and is unable to complete a task that they intended to perform. In this way, the warning signs for memory loss can be made apparent at an early stage.

There is thus provided an improved method and apparatus for monitoring a subject, which overcomes the existing problems.

### Brief Description of the Drawings

For a better understanding of the invention, and to show more clearly how it may be carried into effect, reference will now be made, by way of example only, to the accompanying drawings, in which:
Figure 1 is a block diagram of an apparatus according to an embodiment; and
Figure 2 is a flow chart illustrating a method according to an embodiment.

### Detailed Description of the Preferred Embodiments

As noted above, the invention provides an improved method and apparatus for monitoring a subject, which overcomes the existing problems.

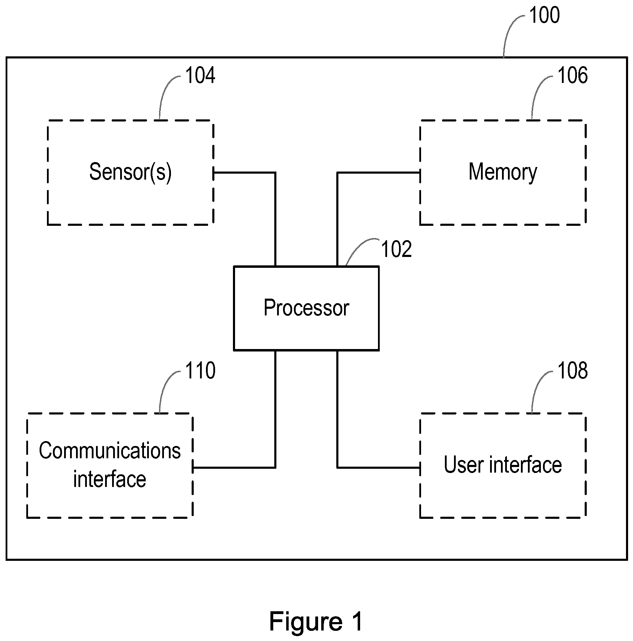

**Figure 1** shows a block diagram of an apparatus 100 according to an embodiment that can be used for monitoring a subject according to an embodiment.

With reference to Figure 1, the apparatus 100 comprises a processor 102 that controls the operation of the apparatus 100 and that can implement the method described herein. The processor 102 can comprise one or more processors, processing units, multi-core processors or modules that are configured or programmed to control the apparatus 100 in the manner described herein. In particular implementations, the processor 102 can comprise a plurality of software and/or hardware modules that are each configured to perform, or are for performing, individual or multiple steps of the method according to embodiments of the invention.

In some embodiments, the apparatus 100 can be a server at a centralised location. In other embodiments, the apparatus 100 may be a device located in the environment of the subject. In other embodiments, the apparatus 100 may be a wearable device configured to be worn by the subject.

Briefly, the processor 102 of the apparatus 100 is configured to acquire from at least one sensor a sequence of sensor signals associated with an environment of a subject and compare the acquired sequence of sensor signals to at least one stored sequence of sensor signals. The stored sequence of sensor signals comprise one or more features associated with an activity. The processor 102 is also configured to identify a stored sequence of sensor signals comprising one or more features that match features of the acquired sequence of sensor signals and detect whether the subject performs the activity associated with the one or more features of the identified sequence of sensor signals. Here, a failure of the subject to perform the activity is indicative of a memory loss event. The processor 102 is also configured to output a notification (or alert) where the subject fails to perform the activity.

In the illustrated embodiment of Figure 1, the apparatus 100 comprises one or more sensors 104, which may comprise at least one sensor operable to acquire a sequence of sensor signals associated with an environment of a subject. However, it will be understood that one or more of sensors operable to acquire a sequence of sensor signals associated with an environment of a subject may alternatively or additionally be external to (i.e. separate to or remote from) the apparatus 100.

In one example, a wearable device configured to be worn by the subject can comprise one or more of the at least one sensors. In embodiments where the apparatus 100 is itself a wearable device, this may be the same wearable device or a separate wearable device. The device comprising one or more of the at least one sensors can be configured to be worn on or around the neck of the subject (e.g. the device may be in the form of a pendant configured to be worn on a cord, chain, necklace, or collar around the neck of the subject) or any other part of the subject suitable for the sensors to acquire a sequence of signals associated with an environment of the subject. In these embodiments, the one or more sensors of the wearable device may be positioned to face outwardly from the subject when the device is worn by the subject to point toward the nominal forward viewing direction of the subject. In another example, the wearable device may be a pair of smart glasses comprising one or more of the at least one sensors. For example, one or more sensors may be embedded in one or both lenses of the smart glasses and positioned to face outwardly from the subject. Alternatively or in addition, in another example, one or more of the at least one sensors may comprise an attachment (such as a clip) for attaching or mounting the sensor to the subject (such as to clothing worn by the subject).

Alternatively or in addition, one or more of the at least one sensors may be located in the environment of the subject (such as to observe the subject). For example, an object in the environment of the subject may comprise one or more of the at least one sensors, and/or the one or more of the at least one sensors may comprise one or more mounted sensors (such as one or more wall-mounted sensors, one or more floor-mounted sensors, one or more ceiling mounted sensors, or any other mounted sensors), one or more sensors that stand in the environment of the subject, or any other sensors, or any combination of sensors, located in the environment of the subject.

Examples of a sensor that may be operable to acquire a sequence of sensor signals associated with an environment of a subject may include, but are not limited to, a visual (or image or spectral) sensor, an acoustic (or audio) sensor, a motion sensor, an electromagnetic field or radiation sensor, or any other sensor suitable to acquire a sequence of sensor signals associated with an environment of a subject. Thus, examples of sensor signals that may be acquired comprise any one or more of visual signals (such as images, or any other visual signals), acoustic signals (such as sounds, or any other acoustic signals), motion signals (such as acceleration signals, or any other motion signals), electromagnetic field or radiation signals, or any other sensor signals, or any combination of sensor signals, associated with an environment of a subject.

A visual (or image or spectral) sensor may be any type of sensor, or any combination of sensors, suitable to acquire a sequence of visual signals (such as images, or any other visual signals) associated with an environment of a subject. Examples of a visual sensor include, but are not limited to, a camera (such as a camera that operates in the visible part of the spectrum, or any other camera), a near-infrared image sensor, or any other visual sensor, or any combination of visual sensors. An acoustic sensor may be any type of sensor, or any combination of sensors, suitable to acquire acoustic signals (such as sounds, or any other acoustic signals) associated with an environment of a subject. Examples of an acoustic sensor include, but are not limited to, a microphone, an ultrasound sensor, or any other acoustic sensor, or any combination of acoustic sensors.

A motion (or activity or inertial) sensor may be any type of sensor, or any combination of sensors, suitable to acquire a sequence of motion signals (such as acceleration signals, or any other motion signals) associated with an environment of a subject. Examples of a motion sensor include, but are not limited to, an accelerometer, a gyroscope, a magnetometer, a visual sensor, a pressure sensor, wi-fi field monitoring sensors, or any other motion sensor, or any combination of motion sensors. An electromagnetic field or radiation sensor may be any type of sensor, or any combination of sensors, suitable to monitor or detect changes in an electromagnetic field or radiation. Examples of an electromagnetic field or radiation sensor include, but are not limited to, a microelectromechanical systems, MEMS, based magnetic field sensor, an antenna (such as an electromagnetic field, EMF, meter), a compass, a magnetometer, or any other electromagnetic field or radiation sensor, or any combination of electromagnetic field or radiation sensors.

Although some examples have been provided for the type of the at least one sensor and the arrangement of the at least one sensor, it will be understood that other types of sensor or combinations of sensors and sensor arrangements are also possible.

In some embodiments, the apparatus 100 may also comprise a memory 106 configured to store program code that can be executed by the processor 102 to perform the method described herein. Alternatively or in addition, one or more memories 106 may be external to (i.e. separate to or remote from) the apparatus 100. For example, one or more memories 106 may be part of another device. A memory 106 can be used to store information, data, signals and measurements acquired or made by the processor 102 of the apparatus 100 or from any components, units, interfaces, sensors, memories or devices that are external to the apparatus 100. Thus, for example, a memory 106 may be used to store at least one sequence of sensor signals or any other information resulting from the method disclosed herein. The processor 102 may be configured to control a memory 106 to store the information resulting from the method disclosed herein.

According to some embodiments, the apparatus 100 may also comprise at least one user interface 108. Alternatively or in addition, a user interface 108 may be external to (i.e. separate to or remote from) the apparatus 100. For example, the user interface 108 may be part of another device.

A user interface 108 may be for use in providing a user of the apparatus 100 with information resulting from the method according to the invention. A user can be any user of the apparatus 100. For example, a user may be a healthcare provider, a healthcare specialist, a caregiver, a family member, the subject themselves, or any other user. The processor 102 may be configured to control one or more user interfaces 108 to provide information resulting from the method according to the invention. For example, in some embodiments, the processor 102 may be configured to control one or more user interfaces 108 to render (or output) the notification. A user interface 108 may, alternatively or in addition, be configured to receive a user input. In other words, a user interface 108 may allow the user of the apparatus 100 to manually enter data, instructions, or information. The processor 102 may be configured to acquire the user input from one or more user interfaces 108.

A user interface 108 may be any user interface that enables rendering (or outputting) of information, data or signals to a user of the apparatus 100. Alternatively or in addition, a user interface 108 may be any user interface that enables a user of the apparatus 100 to provide a user input, interact with and/or control the apparatus 100. For example, the user interface 108 may comprise one or more switches, one or more buttons, a keypad, a keyboard, a mouse, a touch screen or an application (for example, on a smart device such as a tablet or smartphone), a display screen, a graphical user interface (GUI) or any other visual component, one or more speakers, one or more microphones or any other audio or acoustic component, one or more lights, a component for providing tactile feedback (such as a vibration function), or any other user interface, or combination of user interfaces. In some embodiments, the user interface that is controlled to render (or output or display) information, data or signals to the user of the apparatus 100 may be the same user interface as that which enables the user to provide a user input, interact with and/or control the apparatus 100.

Returning back to Figure 1, in some embodiments, the apparatus 100 may also comprise a communications interface (or circuitry) 110 for enabling the apparatus 100 to communicate with (or connect to) any components, interfaces, units, memories, sensors and devices that are internal or external to the apparatus 100. The communications interface 110 may communicate with any components, interfaces units, sensors and devices wirelessly or via a wired connection. For example, in embodiments where one or more sensors 104 are external to the apparatus 100, the communications interface 110 may communicate with the external sensors wirelessly or via a wired connection. Similarly, in embodiments where one or more memories 106 are external to the apparatus 100, the communications interface 110 may communicate with the external memories wirelessly or via a wired connection. Similarly, in the embodiments where one or more user interfaces 108 are external to the apparatus 100, the communications interface 110 may communicate with the external user interfaces wirelessly or via a wired connection.

It will be appreciated that Figure 1 only shows the components required to illustrate this aspect of the invention, and in a practical implementation the apparatus 100 may comprise additional components to those shown. For example, the apparatus 100 may comprise a battery or other power supply for powering the apparatus 100 or means for connecting the apparatus 100 to a mains power supply.

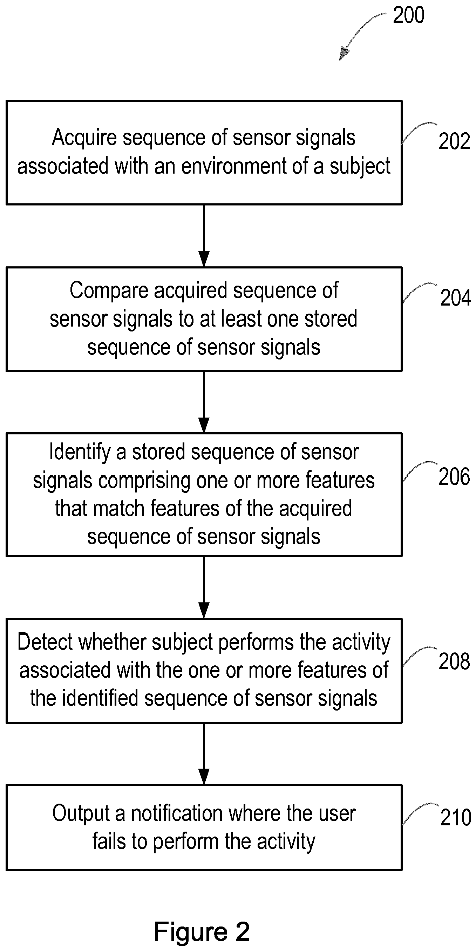

**Figure 2** illustrates a method 200 for monitoring a subject according to an embodiment. The illustrated method 200 can generally be performed by or under the control of the processor 102 of the apparatus 100.

Although not illustrated in Figure 2, in any of the embodiments disclosed herein, the method may initially comprise a calibration (or registration) phase. In some embodiments, for example, the method may comprise storing at least one sequence of sensor signals in a calibration phase. The processor 102 may control one or more memories 106 (which may be one or more memories of the apparatus 100, one or more memories external to the apparatus 100 or both) to store at least one sequence of sensor signals.

The stored sequence of sensor signals comprise one or more features associated with an activity. The activity may comprise one or more tasks (for example, a single task or a set of tasks). The activity can be any activity that may be performed by the subject. In some embodiments, the activity may involve a location of the subject (such as a kitchen, a hallway, a living room, a bathroom or any other location of the subject), one or more objects (such as keys, a coat, a kettle, a knife, a cooker, or any other object), or any other features, or any combination of features, associated with the activity. Thus, the one or more features of the at least one stored sequence of sensor signals can be indicative of any one or more of a location of the subject (such as reference routes for the subject) in the environment that is associated with the activity, one or more objects in the environment that are associated with the activity, or any other features, or any combination of features, that are associated with the activity. In embodiments in which one or more features of the stored at least one sequence of sensor signals are indicative of objects in the environment of the subject associated with the activity, the objects in the environment of the subject may be tagged for this purpose.

In the calibration phase, the at least one sequence of sensor signals can be acquired from at least one sensor (such as any of those described earlier) for subsequent storage and analysis. In some embodiments, at least one sensor may be controlled by the processor 102 of the apparatus to acquire a sensor signal at certain time intervals (for example, regular time intervals). Alternatively or in addition, in some embodiments, the subject or another user may control at least one sensor to acquire at least one sequence of sensor signals from at least one sensor. For example, the subject or user may provide an instruction via a user interface 108 (which may be a user interface of the apparatus 100 or a user interface external to the apparatus 100) for at least one sensor to acquire at least one sequence of sensor signals.

In one example, a sequence of images may be acquired from at least one visual sensor. Alternatively or in addition, in another example, a sequence of sound samples may be acquired from at least one audio or acoustic sensor. A sequence of sound samples may be useful since, for example, the sound of a subject walking can be different in different locations (such as in rooms with different floors or acoustics) and thus, in some embodiments, can be used to define a path to an object. Alternatively or in addition, in another example, a sequence of motion signals may be acquired from at least one motion sensor. In one example embodiment, the subject or user may capture a sequence of images by wearing a device comprising a camera and pressing an "add to list" option on a smartphone app in communication with the device at certain time intervals as the subject or user performs tasks associated with an activity.

Once uploaded to the processor 102, the acquired sequence of sensor signals can be processed to build a timeline associating one or more features (such as pre-defined locations associated with the activity, pre-defined objects associated with the activity, or any other features, or any combination of features) in the acquired sequence of sensor signals to a pre-defined activity. In this way, for each pre-defined activity, the processor 102 can train a specific model that is capable of predicting one or more features that will be present in an acquired sequence of sensor signals for a pre-defined activity.

As mentioned previously, the one or more features of the stored at least one sequence of sensor signals can be indicative of any one or more of a location of the subject in the environment and an object in the environment of the subject. However, the one or more features may alternatively or in addition be indicative of other factors that may influence the subject. Thus, a more complete list of examples for the one or more features includes, but is not limited to, any one or more of the acquired sensor signal itself (for example, a measured sensor signal, which may be a raw sensor signal), a low-level feature characterising one or more aspects of the sensor signal (for example, a statistical variance of the sensor signal or any other low-level feature), an object in the environment of the subject, a location of the subject in the environment, a movement of the subject in the environment, a posture of the subject in the environment, a time of day in the environment of the subject, the weather in the environment of the subject, the number of people in the environment of the subject, or any other features, or any combination of features, associated with an activity.

The one or more features themselves can also be stored in the calibration phase. In any of the embodiments disclosed herein, one or more features can be added, removed (for example, when a feature is no longer needed), or both added and removed to the stored features. In some embodiments, the one or more features can be stored with an image of the feature. For example, in one example embodiment, the subject or user may capture an image of the feature (for example, an object) by wearing a device comprising a camera and pressing an "add to list" option on a smartphone app in communication with the device to store the image of the feature.

When an image of a feature is stored, in some embodiments, the processor 102 of the apparatus 100 can be configured to automatically learn routes to find the feature. For example, images may be taken by a camera at regular time intervals and a recent history of these images may be stored in a memory. Each stored picture can be searched for predefined features (for example, objects). Once a feature is recognised in an image, the sequence of images acquired in the time interval before the image in which the feature is recognised are stored (or encoded). In this way, the route of the subject to the object can be recorded. In one example, a route may start when the subject gets up from a chair, walks to the corridor and picks up their coat. Since many routes can lead to a common object, in some embodiments, a plurality of different sequences of images may be stored with an object.

In some embodiments, the subject or another user may have permission to review stored sequences of signals and any corresponding information. For example, for each sequence of images, the subject or another user may be allowed to indicate whether an activity associated with the sequence of signal is to be classed as normal or abnormal for that sequence of images. An example of this is that the activity of cooking associated with a sequence of signals associated with the environment of a kitchen is classed as normal.

In some embodiments, the one or more features of the stored sequence of sensor signals are each stored with a weight indicative of the significance (or importance) of the feature compared to the other features. The weight may be a pre-set weight or may be programmable by a user. Alternatively, the weight may be determined based on changes in one or more physiological signals that are acquired for the subject during the calibration phase. For example, if forgetting the location of glasses leads to a higher observed heart rate and a lower heart rate variability (which can be indicative of stress) of the subject than forgetting the location of other objects, this feature may be assigned a higher weight.

As the significance of a feature can be personal and can also vary over time, the weight may be adaptable to a user. Where one or more features are added to the stored features, an associated weight indicative of the significance (or importance) of the feature may also be added. In some embodiments, the processor 102 may perform more computations to search for features in acquired sensor signals that have a higher weight than those features that have a lower weight. Alternatively or in addition to the weight, the one or more features of the stored sequence of sensor signals are each stored with an a priori probability of the subject failing to perform the activity associated with the one or more features.

In some embodiments, the one or more features of the stored sequence of sensor signals may be stored in the form of a look-up table. An example of such a look-up table is provided below:

| **Feature** | **Type** | **Weight** | **Probability of failure** |
|---|---|---|---|
| **Key of home** | Object | 5 | 5 |
| **Hearing-aid** | Object | 5 | 5 |
| **Glasses** | Object | 5 | 5 |
| **Likely home key locations** | Location | 5 | 5 |
| **Rain-coat location (e.g. entrance)** | Location | 1 | 1 |
| **Kitchen** | Location | 3 | 1 |

In this example, the features are assigned a weight between 1 and 5 where 1 indicates a feature of the lowest significance and 5 indicates a feature of the highest significance. Similarly, in this example the a priori probability of the subject failing to perform the activity associated with the one or more features is between 1 and 5 where 1 indicates the lowest probability of the subject failing to perform the activity and 5 indicates the highest probability of the subject failing to perform the activity. Although not illustrated in the example table, the features may also be stored with a specific user to contact in case of a memory failure event. For example, depending on the memory failure event, different users may be notified such as a healthcare provider, a healthcare specialist, a caregiver, a family member, the subject themselves, or any other user.

The information (such as the weight, the probability of failure, or any other information, or any combination of information) associated with the one or more features can be dynamically updated over time. For example, in some embodiments, the information associated with the one or more features can be dynamically updated over time based on the performance of the subject in performing activities, based on changes in one or more physiological signals acquired for the subject, or based on both the performance of the subject in performing activities and changes in one or more physiological signals acquired for the subject.

In any of the embodiments disclosed herein, the one or more features of the stored sequence of sensor signals may be associated with a risk level for the subject and the notification that is output can be based on the risk level associated with the one or more features of the identified sequence of sensor signals. In some embodiments, the risk level can be manually input (for example, via one or more user interfaces 108). Alternatively or in addition, the risk level can be predefined based on the one or more features of the stored sequence of sensor signals (such as the location of the subject in the environment, the objects in the environment of the subject, or any other feature, or any combination of features). For example, where the one or more features comprise a cooker as an object in the environment of the subject, the risk level associated with this feature may be set higher than other features given that an incomplete activity associated with a cooker can be potentially dangerous. In some embodiments, the one or more features of the stored sequence of sensor signals may be associated with an alert setting based on the risk level associated with those features, where different features may have different alert settings.

With reference to Figure 2, at block 202, a sequence of sensor signals associated with the environment of the subject is acquired from at least one sensor. For example, the processor 102 may be configured to control at least one sensor to acquire the sequence of sensor signals. The sequence of sensor signals may be acquired from any one or more of the sensors described earlier. Thus, the sensor signals may comprise any one or more of visual signals (such as images), audio or acoustic signals (such as sounds), motion signals, or any other sensor signals, or any combination of sensor signals associated with an environment of a subject.

At block 204 of Figure 2, the acquired sequence of sensor signals is compared to the at least one stored sequence of sensor signals. As described earlier, the at least one stored sequence of sensor signals to which the acquired sequence of sensor signals is compared may be stored in one or more memories 106, which may comprise one or more memories of the apparatus 100, one or more memories external to the apparatus 100, or both. As also described earlier, the stored sequence of sensor signals comprise one or more features associated with an activity. The one or more features can, for example, comprise any one or more of the features mentioned earlier, or any other features, or any combination of features associated with the activity.

At block 206 of Figure 2, a stored sequence of sensor signals comprising one or more features that match features of the acquired sequence of sensor signals is identified. By identifying a stored sequence of sensor signals comprising one or more features that match features of the acquired sequence of sensor signals, an activity that the subject intends to perform can be identified as the activity associated with the one or more features of the identified sequence of sensor signals. For example, where the acquired sequence of sensor signals is found to have more features in common with a stored sequence of sensor signals than with other stored sequences of sensor signals, it can be determined that it is the intention of the subject to perform the particular activity that is linked to the features of that stored sequence.

In an example embodiment, the acquired sequence of sensor signals may be correlated (or matched) directly with each stored sequence of sensor signals. In this example embodiment, the stored sequence of sensor signals for which the correlation (or match) is highest can be identified as best matching or most similar. In certain cases, it may be that none of the stored sequences of sensor signals sufficiently match the acquired sequence of sensor signals, in which case no match is found. In this case, information such as the acquired sequence of sensor signals may be stored for later review by a user. Where the acquired sequence of sensor signals matches a stored sequence of sensor signals sufficiently, it may be determined that the activity associated with that stored sequence of sensor signals is being performed, as will be explained in more detail later. Since multiple stored sensor signals form a sequence associated with an entire (or completed) activity, it can be detected whether the full activity has been completed by determining whether the entire sequence of signals is detected.

In an embodiment in which the one or more features comprise at least one object, an activity may be defined as observing the at least one object along a route of the activity. In this embodiment, a stored sequence of sensor signals can be associated with a plurality of object (e.g. table, coat, or any other object) images for the activity. The acquired sequence of sensor signals may then be searched specifically for the object images. If the object is found, a correct match is identified and it is determined that the subject is performing the particular activity associated with the stored sequence of sensor signals for which the correct match is identified.

In some embodiments, the classification algorithm described above with reference to blocks 204 and 206 of Figure 2 may be performed from the moment the start of an activity is detected onwards.

Thus, as described above, a stored sequence of sensor signals comprising one or more features that match features of the acquired sequence of sensor signals can be identified. As the identified sequence of sensor signals is associated with an activity (which may comprise one or more tasks, as mentioned earlier), it is possible to automatically detect that the subject intended to perform the activity. Then, it is detected whether the subject has difficulty performing the activity due to memory problems.

At block 208 of Figure 2, it is detected whether the subject performs the activity associated with the one or more features of the identified sequence of sensor signals. For example, the acquired sequence of sensor signals can be analysed to detect whether the subject performs the activity associated with the one or more features of the identified sequence of sensor signals or whether the subject fails to perform (or fails to complete) the activity.

A failure of the subject to perform (or complete) the activity is indicative of a memory loss event. In this way, it is detected whether the subject has difficulty performing the activity due to memory problems. In some embodiments, a memory loss event can be detected in real-time or at least in near real-time. For example, this may be useful in providing the subject with prompt or immediate assistance. In some cases, a real-time detection may not be necessary and thus, in other embodiments, the detection may occur at a later time. For example, a detection may be made at a later time where the detection is for use in the analysis of memory loss events (such as determining the number of memory loss events that occurred, the type of the memory loss events that occurred, or similar).

Examples of the subject failing to perform (or complete) an activity include, but are not limited to, the subject not moving more than a minimum amount for a predefined time interval, the subject moving (for example, walking) to a different location (for example, back to a previous location), the subject beginning to move (for example, walk around) without completing one or more tasks associated with the activity (for example, in the case of the subject moving to the location of the kitchen, this may comprise the subject not touching a water tap, not beginning to cook, not retrieving an object from a refrigerator, or similar), the subject not completing all of the tasks that logically belong to the activity (for example, the subject turning on a stove without placing a pan on the stove within a predefined time interval). Where the activity involves an object, it may be detected that the subject performs the activity where the object is moved, taken away from the current location, used (such as in the case of an appliance or light switch), or similar. Otherwise, it may be detected that the subject fails to perform the activity.

In some embodiments, where it is not possible to identify a full stored sequence of sensor signals comprising one or more features that match features of the acquired sequence of sensor signals (at block 206), a notification may be output to a user other than the subject. Alternatively or in addition, in one example embodiment, if the first three quarters of a stored sequence of sensor signals is matched to the acquired sequence of sensor signals with sufficient confidence (or correlation), it may be determined that three quarters of the activity has been performed (at block 208). Then, if within a given duration (for example, 5 minutes, or any other duration, which may depend on the activity) the remaining one quarter of the sequence of sensor signals associated with the activity is not matched, it may be detected that the activity is not successfully complete and thus that the subject fails to perform the activity.

At block 210 of Figure 2, where the subject fails to perform the activity, a notification (or alert) is output (or rendered). The notification may be output (or rendered) via one or more user interfaces 108, which may comprise one or more user interfaces of the apparatus 100, one or more user interfaces external to the apparatus, or both. In some embodiments, the processor 102 may be configured to control the one or more user interfaces 108 to output (or render) the notification. The notification can be output to any one or more of the subjects to assist the subject in performing the activity and another user to indicate the subject failing to perform the activity (for example, such that the user can assist the subject).

In some embodiments, the notification can comprise an indication that a memory failure event has occurred. Alternatively or in addition, the notification can comprise information on which (or which types of) locations, objects, or both are involved. In some embodiments, the notification may comprise an identified location of the subject. In some embodiments, the notification may be in the form of sound. For example, one or more user interfaces 108 comprising at least one speaker can be controlled to output assistance to the subject through voice commands. In some embodiments, the notification may be in the form of a visual notification. For example, one or more user interfaces 108 comprising at least one display can be controlled to display to the subject one or more images of objects that represent logical next steps for completing the activity. For example, where the subject has turned on a stove, images of pans and food may be displayed to help the subject progress with the cooking activity. In this way, a memory failure event (and optionally also information or details regarding the memory failure event) can be communicated to the subject, one or more other users, or both.

Although not illustrated in Figure 2, in any of the embodiments disclosed herein, the method may further comprise detecting whether the subject is moving to perform at least part of the activity (for example, via a motion sensor of a device worn by the subject) and, where the subject fails to move to perform at least part of the activity for a minimum period of time, the notification may be output to a user other than the subject. In some embodiments, the minimum period of time may be set based on the one or more features of the identified sequence of sensor signals. In one example, the start of an activity or motion of the subject may initially be detected. Then, it may be detected that the subject has arrived at a predefined location. If the subject fails to move to perform at least part of the activity for a minimum duration, then the notification is output to a user other than the subject. In some embodiments, this notification may comprise information on the location of the subject and the duration of the failure of the subject to move.

Although also not illustrated in Figure 2, in any of the embodiments disclosed herein, the method may further comprise acquiring from at least one physiological sensor one or more physiological signals for the subject. Examples of a physiological sensor include, but are not limited to, a heart rate sensor configured to acquire a signal indicative of a heart rate of the subject, a heart rate variability sensor configured to acquire a signal indicative of a heart rate variability of the subject, a skin conductance sensor configured to acquire a signal indicative of a skin conductance response of the subject, a skin temperature sensor configured to acquire a signal indicative of a skin temperature of the subject, an audio or acoustic sensor configured to acquire a signal indicative of speech of the subject, a visual sensor configured to acquire a signal indicative of a change in facial expressions of the subject, a posture sensor configured to acquire a signal indicative of a body posture of the subject, a gait sensor configured to acquire a signal indicative of a gait of the subject, or any other physiological sensor, or any combination of physiological sensor.

In embodiments where one or more physiological signals for the subject are acquired, the method also comprises determining a likelihood that the failure of the subject to perform the activity is due to the memory loss event based on the acquired physiological signals. In some embodiments, the likelihood of the failure being due to the memory loss event can be determined based on a model of a variation in one or more physiological signals prior to, during, and after a memory loss event. The model can be machine learnt for use in supporting the detection of memory loss events.

In one example, training data is first collected and labelled over time based on features extracted from one or more physiological signals. The training data may be collected from the subject themselves and/or from one or more similar subjects. A memory loss event is detected and verified by a user (such as a healthcare provider, a healthcare specialist, a caregiver, a family member, the subject themselves, or any other user). Once a memory loss event is detected and verified, the one or more physiological signals associated with the memory loss event are stored and labelled to indicate a true memory loss event. In this way, representative training data is made available. The representative training data can be used to train binary classifiers, which can output whether a memory loss event is happening based on one or more acquired physiological signals, and the corresponding likelihood that the memory loss event is happening. The use of physiological signals to detect a memory loss event can be beneficial in detecting activities that are associated with new and unregistered objects or locations. Where a new and unregistered object or location is detected, an alert may be provided to a user for the user to register the object or location.

In this way, using physiological signals, the confidence in a reliable determination of the failure of the subject to perform the activity being due to a memory loss event can be higher. In some embodiments, the determined likelihood of the failure being due to the memory loss event can be used to determine whether to output a notification. In this way, the number of false alarms can be reduced.

Although also not illustrated in Figure 2, in any of the embodiments disclosed herein, the method may further comprise determining a severity of one or more memory loss events over time. For example, the severity of the one or more memory loss events over time may be based on the frequency of occurrence of the one or more memory loss events over time. The determined severity of one or more memory loss events over time may be used to select a user to notify (for example, a healthcare provider, a healthcare specialist, a caregiver, a family member, the subject themselves, or any other user), to adapt the information comprised in the notification that is output (such as the type of location, the type of objects, or both), to modify a frequency and/or strength of the notification that is output, to adapt the stored weights indicative of the significance of a feature and/or the stored probabilities of the subject failing to perform an activity, or any combination of these.

There is therefore provided an improved method and apparatus for monitoring a subject. The method and apparatus described herein can be particularly useful for monitoring subjects that experience memory problems such as dementia and helping or assisting the subject to perform activities. The subject can be supported to perform activities either by providing the subject with direct assistance or by signalling to another user (for example, a healthcare provider, a healthcare specialist, a caregiver, a family member, or any other user) that the subject requires assistance.

There is also provided a computer program product comprising a computer readable medium, the computer readable medium having computer readable code embodied therein, the computer readable code being configured such that, on execution by a suitable computer or processor, the computer or processor is caused to perform the method or methods described herein.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A method (200) for monitoring a subject, the method comprising:
acquiring (202) from at least one sensor a sequence of sensor signals associated with an environment of a subject;
comparing (204) the acquired sequence of sensor signals to at least one stored sequence of sensor signals, wherein the stored sequence of sensor signals comprise one or more features associated with an activity;
identifying (206) a stored sequence of sensor signals comprising one or more features that match features of the acquired sequence of sensor signals;
detecting (208) whether the subject performs the activity associated with the one or more features of the identified sequence of sensor signals, wherein a failure of the subject to perform the activity is indicative of a memory loss event; and
outputting (210) a notification where the subject fails to perform the activity.

2. A method as claimed in claim 1, wherein the sensor signals comprise any one or more of visual signals, acoustic signals and motion signals.

3. A method as claimed in claim 1 or 2, wherein the features comprise any one or more of:
an acquired sensor signal;
a low-level feature characterising one or more aspects of the sensor signal;
an object in the environment of the subject;
a location of the subject in the environment;
a movement of the subject in the environment;
a posture of the subject in the environment;
a time of day in the environment of the subject;
the weather in the environment of the subject; and
the number of people in the environment of the subject.

4. A method as claimed in any one of the preceding claims, wherein the one or more features of the stored sequence of sensor signals are each stored with any one or more of:
a weight indicative of the significance of the feature compared to the other features; and
an a priori probability of the subject failing to perform the activity associated with the one or more features.

5. A method as claimed in any one of the preceding claims, wherein the notification is output to any one or more of:
the subject to assist the subject in performing the activity; and
another user to indicate the subject failing to perform the activity.

6. A method as claimed in any one of the preceding claims, wherein the one or more features of the stored sequence of sensor signals are associated with a risk level for the subject and the notification that is output is based on the risk level associated with the one or more features of the identified sequence of sensor signals.

7. A method as claimed in any one of the preceding claims, the method further comprising:
acquiring from at least one physiological sensor one or more physiological signals for the subject; and
determining a likelihood that the failure of the subject to perform the activity is due to the memory loss event based on the acquired physiological signals.

8. A method as claimed in any one of the preceding claims, the method further comprising:
determining a severity of one or more memory loss events over time.

9. A method as claimed in any one of the preceding claims, the method further comprising:
detecting whether the subject is moving to perform at least part of the activity; and
where the subject fails to move to perform at least part of the activity for a minimum period of time, outputting the notification to a user other than the subject.

10. A method as claimed in any one of the preceding claims, wherein the method further comprises:
storing the at least one sequence of sensor signals in a calibration phase,
wherein the one or more features of the at least one stored sequence of sensor signals are indicative of any one or more of:
a location of the subject in the environment that is associated with the activity; and
one or more objects in the environment that are associated with the activity.

11. A computer program product comprising a computer readable medium, the computer readable medium having computer readable code embodied therein, the computer readable code being configured such that, on execution by a suitable computer or processor, the computer or processor is caused to perform the method of any one of claims 1-10.

12. An apparatus (100) for monitoring a subject, the apparatus (100) comprising:
a processor (102) configured to:
acquire from at least one sensor (104) a sequence of sensor signals associated with an environment of a subject;
compare the acquired sequence of sensor signals to at least one stored sequence of sensor signals, wherein the stored sequence of sensor signals comprise one or more features associated with an activity;
identify a stored sequence of sensor signals comprising one or more features that match features of the acquired sequence of sensor signals;
detect whether the subject performs the activity associated with the one or more features of the identified sequence of sensor signals, wherein a failure of the subject to perform the activity is indicative of a memory loss event; and
output a notification where the subject fails to perform the activity.

13. An apparatus (100) as claimed in claim 12, wherein the at least one sensor (104) comprises any one or more of a visual sensor, an acoustic sensor, a motion sensor, an electromagnetic field, and a radiation sensor.

14. An apparatus (100) as claimed in claim 12 or 13, wherein the apparatus (100) comprises one or more of the at least one sensors (104).

15. An apparatus (100) as claimed in any one of claims 12, 13 or 14, wherein:
a wearable device configured to be worn by the subject comprises one or more of the at least one sensors (104);
an object in the environment of the subject comprises one or more of the at least one sensors (104); and/or
one or more of the at least one sensors (104) are located in the environment of the subject.
